# EUROPEAN PATENT SPECIFICATION

(11) **EP 3 689 417 B1**
(45) Date of publication and mention of the grant of the patent: **07.06.2023**
(21) Application number: 19192352.3
(22) Date of filing: 19.08.2019
(51) Int. Cl.: H01R 24/50, A61N 1/375, H01M 50/503, H01M 50/519, H01R 12/57, H01R 13/627, H01R 24/38, H01R 13/11, H01R 103/00

(54) **IMPLANTABLE MEDICAL DEVICE COMPRISING A CONNECTOR ARRANGEMENT FOR ESTABLISHING A TWO-POLE ELECTRIC CONTACT BETWEEN COMPONENTS**
IMPLANTIERBARE MEDIZINISCHE VORRICHTUNG MIT EINER VERBINDERANORDNUNG ZUR HERSTELLUNG EINES ZWEIPOLIGEN ELEKTRISCHEN KONTAKTS ZWISCHEN KOMPONENTEN
DISPOSITIF MÉDICAL IMPLANTABLE AVEC UN AGENCEMENT DE CONNECTEUR POUR ÉTABLIR UN CONTACT ÉLECTRIQUE BIPOLAIRE ENTRE DES COMPOSANTS

(30) Priority: 30.01.2019 EP 19154499
(43) Date of publication of application: 05.08.2020
(73) Proprietor: BIOTRONIK SE & Co. KG, 12359 Berlin (DE)
(72) Inventor: Henschel, Martin, 13125 Berlin (DE); Hartmann-Bax, Kathy, 14947 Nuthe-Urstromtal (DE); Oertmann, Torsten, 15827 Blankenfelde (DE)
(74) Representative: Biotronik Corporate Services SE

(56) References cited:
- US-A- 4 743 205
- US-A- 5 103 818
- US-A- 5 791 911

## Description

The present disclosure relates to an implantable medical device comprising a connector arrangement for establishing a two-pole electric contact between components of the implantable medical device.

### BACKGROUND

Nowadays, components of implantable medical devices are usually provided with adapters for enabling an electrical connection, e.g., between a battery or a capacitor and a functional component of an implantable medical device. In most cases, components such as a battery or a capacitor, have two potentials which need to be connected: a plus pole (cathode), which may be contacted, e.g., via a feedthrough pin, and a minus pole (anode), which may be contacted, e.g., via a further pin or an adapter, or directly at a housing of the battery or capacitor. Frequently, the housing has the minus potential. Conventionally, adapters are provided on the plus and/or minus pole, such that a two-pole electric contact with a functional component of the implantable medical device may be achieved by welding, soldering, or brazing on the adapters.

The document US 2016/0315302 A1 discloses an implantable medical device comprising a battery housing, a feedthrough member extending from the battery housing, and a connector including at least one electrical terminal electrically communicating with the feedthrough member.

The document US 2007/0150020 A1 describes a battery for use with implantable medical devices, the battery including a battery housing, a connector block connected to the battery housing, a feedthrough assembly having a ferrule, wherein at least a portion of the ferrule extends outside the battery housing, and within the connector block.

Documents US 5791911 and US 4743205 disclose coaxial electrical connectors.

It is a drawback of the known solutions that additional adapters need to be arranged on the components, such as on the battery, capacitor and/or the functional component of the implantable medical device. Such adapters require space and make the product more expensive. Currently, metallurgical connections (such as welding or soldering) are usually used for the electrical ground contact as well as for the anode contact. The soldering partners or welding partners require a relatively large amount of space on the circuit. Furthermore, the number of contact transitions may be increased by such adapters, yielding increased contact resistances and power losses. As a result, a lifetime of an implant may be shortened.

An exemplary known solution in connection with a battery of an implant functions as follows: a foil is welded on a battery pad. This is the adapter, which is welded on the housing of the battery. One end of a wiring strip is then welded to the adapter. The other end of the wiring strip is welded to a metallic pad, which in turn is soldered to circuitry of the implant. As a result, the following transitions are formed: battery housing - metal foil - battery adapter pad - wiring strip - circuitry adapter pad - circuitry. This amounts to 5 metallurgical transitions at the minus pole and 4 metallurgical transitions at the plus pole, i. e, 9 transitions in the closed electric circuit.

### SUMMARY

It is an object of the present invention to provide an improved connector arrangement for establishing a two-pole electric contact between components of an implantable medical device.

According to claim 1, an implantable medical device is provided, comprising a connector arrangement for establishing a two-pole electric contact between components of the implantable medical device comprises: a first connector portion comprising a first contact element and a pin receptacle; and a second connector portion comprising a second contact element and a contact pin; wherein the connector arrangement is configured to assume a connected state, in which the first contact element is in contact with the second contact element and the contact pin is received in the pin receptacle. At least a part of the first connector portion and/or of the second connector portion is mounted on a printed circuit board (PCB).

In a preferred embodiment, the first contact element and the pin receptacle are mounted on the PCB.

It is proposed to directly connect a connector portion with a PCB instead of using an additional adapter. With this solution, the number of contact transitions may be reduced, e. g, to two contact transitions for each pole. For example, in the connected state, the first contact element may be in contact with the second contact element for connecting a first pole (e.g. the minus pole), and the contact pin may be received in the pin receptacle for connecting a second pole (e.g. the plus pole). In that case, two interconnection transitions at each pole may be sufficient: a transition from, e.g., a battery housing to the first contact element and a transition from the first contact element to the circuitry (minus pole); and a transition from the contact pin to the first connector portion and a transition from the first connector portion to the circuitry (plus pole), yielding in total 4 transitions for the closed electric circuit.

As a result of reducing the number of required metallurgical transitions, the contact resistance and the corresponding power losses may be reduced. In addition, a contact reliability may be enhanced, since fewer process steps are involved in the formation of the interconnection. Further, a space reduction of the interconnection may be achieved. For example, the reduction of the required volume of the interconnections may be larger than 50% as compared to a prior art solution due to the fewer required welding contacts. Furthermore, processing costs may be saved due to a reduction of the processing time required for establishing the interconnections (fewer welding steps). More generally, due to the saving of a dedicated battery adapter, the product costs may be reduced.

In an embodiment, the first connector portion is mounted on the PCB of the implantable medical device, and the second connector portion is arranged on a battery, a capacitor, or a feedthrough assembly of the implantable medical device.

In an embodiment, the first connector portion and/or the second connector portion may form at least a part of a surface-mount device (SMD) that is mounted on the PCB. In other words, a surface-mount technology (SMT) may be used for attaching the first connector portion and/or the second connector portion to the PCB. Thus, an automated assembly may be facilitated, e. g. by allowing for an automated placement of the first connector portion and/or the second connector portion on the PCB. For example, the first connector portion and/or the second connector portion, which is formed as SMD, may thus be automatically soldered to the PCB, e. g. in an automated reflow soldering process.

In an embodiment, the pin receptacle is mounted on a first side of the PCB, whereas the first contact element is mounted on a second side of the PCB that is opposite to the first side of the PCB. In that case, for example, each of the pin receptacle and the first contact element may be soldered to the PCB sequentially by means of a respective reflow soldering process. This is to say that a top side and a bottom side of the PCB may be sequentially fitted with the pin receptacle and the first contact element, respectively.

For example, the first connector portion may comprise a contact pad. The pin receptacle may be formed in the contact pad, e.g., as a hole extending into or through the contact pad.

Further, in an embodiment, the first connector portion comprises at least one guidance element that is configured to align the first contact element and the contact pad. In particular, the guidance element may be configured to axially align the first contact element and the contact that during assembly, e.g., during a reflow soldering process. For example, during the soldering process, when each of the pin receptacle and the first contact element "swim" in the solder for a short while and the solder solidifies during cooling off, there is a risk that an axial offset between these two contact components arises. This offset may be partially compensated for by means of inclined insertion surfaces that may be provided at the contact pin and/or at the pad which includes the pin receptacle, such that the contact pin is guided towards the hole (i.e., the pin receptacle) formed in the contact pad. To further minimize such position tolerances, one or more guidance elements may be arranged at the first contact element and/or at the contact pad so as to ensure an axial alignment by avoiding an unwanted displacement during the reflow process (i.e. while the solder is liquid).

In one embodiment, the connector arrangement is in the connected state and the contact pin is materially bonded with the pin receptacle. For example, the material bond may be a metallurgical bond, such as a welded joint, a soldered joint, or a brazed joint. For example, by fixing the contact pin to the pin receptacle (e.g. to a contact pad forming the pin receptacle) by means of a welding spot or a soldering spot, an extraordinary contact reliability, which is required for medical implants, may be ensured. Further, due to the fixing of the contact pin to the pin receptacle by means of, e.g., a welded joint or a soldered joint, also the first contact element may be held in contact with the second contact element. For example, there may be provided a spring connection or a latching connection (also referred to as snap-in connection) between the first contact element and the second contact element, wherein the fixing of the connection pin and the pin receptacle by means of soldering or welding may secure said spring connection or latching connection of the first contact element and the second contact element. Thus, the welding j oint or soldering joint may secure the connection at both electric poles (plus and minus) at the same time. Further, the pin contact and/or the mechanical contact (e.g., spring contact and/or latching contact) between the first contact element and the second contact element may also facilitate the welding and/or soldering process by positioning the welding or soldering partners relative to each other without requiring a dedicated external tool (in accordance with the so-called "hands-off" principle during the assembly).

In addition, an automated placement as well as an automated contacting of the components of the implantable medical device may be enabled with this connector assembly due to the uniaxial assembly.

Instead of the mentioned welding spots and/or soldering spots, further reliable contacts, such as a crimped contact, an installation displacement contact, and/or a spring contact, are conceivable. More generally, in addition or alternatively to the above-mentioned material bond, the contact pin may be form-fittingly and/or force-fittingly connected to the pin receptacle in the connected state of the connector arrangement. In a preferred embodiment, the first connector portion comprises a first spring element that is configured to establish a form-fitting and/or force-fitting connection between the pin receptacle and the contact pin in the connected state of the connector arrangement.

In one embodiment, the first contact element and the second contact element are configured to be connected with each other by means of a spring connection and/or by means of a snap-in connection (i.e., a latching connection) in the connected state of the connector arrangement. For example, one of the first contact element and the second contact element may comprise at least one second spring element and the other one of the first contact element and the second contact element may comprise at least one groove, wherein the at least one second spring element engages with the at least one groove in the connected state of the connector arrangement.

In one embodiment, the first contact element and/or the second contact element have an annular basic shape. For example, the first contact element may be formed in one piece having an annular basic shape. Alternatively, the first contact element may consist of several pieces, such as two basically semicircular elements.

In the connected state, the first contact element, the second contact element, the contact pin, and the pin receptacle may be arranged coaxially. In particular, each of the first contact element, the second contact element, the contact pin, and the pin receptacle may be arranged coaxially with respect to a common symmetry axis. For example, said common symmetry axis may be defined by each of a main extension axis of the contact pin and a (virtual) axis of a respective annular basic shape of the first contact element and the second contact element. The connector arrangement may be very volume efficient (i.e. space saving) due to such a coaxial arrangement. In particular, it may thus not be necessary to provide contacts that are arranged next to each other. This may again have a positive effect on the production costs, since less contacts need to be welded.

Further, a simple, uniaxial assembly may be achieved in combination with a self-clamping function that may be realized, e. g., by a spring connection between the first contact element and the second contact element and/or by a spring connection between the contact pin and the pin receptacle. The form-fitting and/or force-fitting connections between the first contact element and the second contact element and/or between the connection pin and the pin receptacle may also provide an integrated hold-down and alignment function for a wedding or soldering process which safely connects the pin receptacle and the contact pin. As a result of this self-clamping function, it is not necessary to hold down the components manually or to provide dedicated external tools to this end. A hands-off process may thus be enabled.

In an embodiment, the first connector portion and/or the second connector portion comprises an insulation element, which may, e.g., comprise or consist of a plastic material, such as an insulating mold compound. For example, the first connection element may be mounted on an insulation element.

The invention can be also used to connect the circuit board to one or more feedthrough(s) of the device header, e.g., to transmit and receive electrical signals from the heart leads that are connected to the header or to transmit and receive RF signals from an antenna that is positioned within the header.

All aspects and features of the embodiments described above and in the following can be combined with each other unless explicitly stated otherwise.

### DESCRIPTION OF THE DRAWINGS

The various features and advantages of the present invention may be more readily understood with reference to the following detailed description and the drawings. Herein,
- Fig. 1: shows a perspective view of a connector arrangement;
- Fig. 2: shows a cross-section view of the connector arrangement of Figure 1;
- Fig. 3: shows a cross-section view of the connector arrangement of Figure 1 in a connected state;
- Fig. 4: shows an exploded view of a first connector portion of the connector arrangement of Figure 1 in combination with a PCB;
- Fig. 5: shows a cross-section view of the first connector portion of Figure 4 in an assembled state;
- Fig. 6: shows a perspective view of a first contact element arranged on an insulation element;
- Fig. 7: shows a perspective view of the first contact element of Figure 6;
- Fig. 8: shows a perspective view of another variant of a first contact element;
- Fig. 9: shows a cross-section view of a feedthrough assembly having a second connector portion;
- Fig. 10: shows a cross-section view of the second connector portion of Figure 9 that is connected with a first connector portion;
- Fig. 11: shows a cross-sectional view of another variant of a second connector portion;
- Fig. 12: shows a cross-section view of yet another variant of second connector portion;
- Fig. 13: shows a perspective view of a PCB and a contact pad forming a pin receptacle;
- Fig. 14: shows the other side of the PCB of Figure 13 and a first contact element arranged on an insulation element;
- Fig. 15: shows a close-up perspective view of the insulation element and the first contact element of Figure 14;
- Fig. 16: shows a cross-section view of the PCB, the contact pad, and the insulation element with the first contact element in an assembled state;
- Fig. 17: shows a cross-section view of the first connector portion of Figure 16 connected to a second connector portion of a battery;
- Fig. 18: shows a PCB and a contact pad forming a pin receptacle and having a plastic border;
- Fig. 19: shows the components of Figure 18 and a first contact element arranged on an insulation element;
- Fig. 20: shows the components of Figure 19 is in an assembled state;
- Fig. 21: shows a perspective view of the first connector portion of Figure 20 and a battery having a second connector portion;
- Fig. 22: shows a cross-section view of the connector arrangement of Figure 21 in a connected state;
- Fig. 23: shows a perspective view of a PCB and an array of two contact pads, each forming a respective pin receptacle and having a common plastic border;
- Fig. 24: shows the PCB and the contact pad array of Figure 23 as well as two first contact elements that are arranged on respective insulation elements;
- Fig. 25: shows a cross-section view of the first connector portion of Figure 24 that is connected to a second connector portion of a battery
- Fig. 26: shows a perspective view of a connector arrangement in the connected state;
- Fig. 27: shows the connector arrangement of Figure 26, wherein the contact pin is soldered to the pin receptacle;
- Fig. 28: shows a cross-section view of the connector arrangement of Figure 27;
- Fig. 29: shows another variant of a first connector portion, wherein the pin receptacle is formed by a first spring element;
- Fig. 30: shows a cross-section view of the first connector portion of Figure 29 that is connected with a second connector portion;
- Fig. 31: shows yet another variant of a connector assembly having a spring connection for securing the contact pin in the connected position; and
- Fig. 32: shows a cross-section view of the connector assembly of Figure 31.

### DETAILED DESCRIPTION

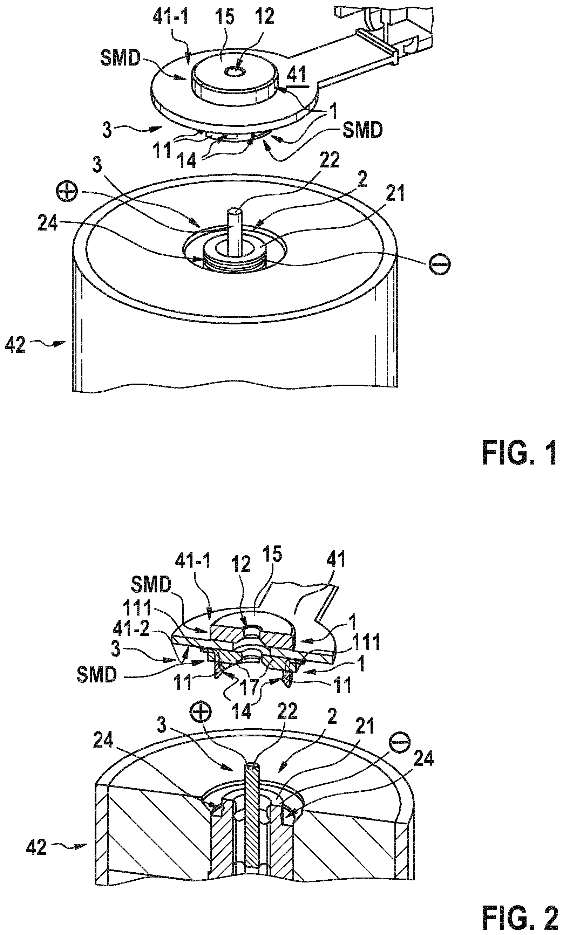

Figure 1 shows a perspective view of a connector arrangement 3 in accordance with the invention. The connector arrangement 3 is provided for establishing a two-pole electric contact between a battery 42 of an implantable medical device and another component, such as an electronics module of the implantable medical device. For example, the implantable medical device may be a pacemaker, an ICD (ICD - implantable cardioverter-defibrillator), a neuro stimulator, e.g. for spinal cord stimulation, a loop recorder, a sensor, such as a pressure sensor, or a leadless pacemaker.

The connector arrangement 3 comprises a first connector portion 1 and a second connector portion 2.

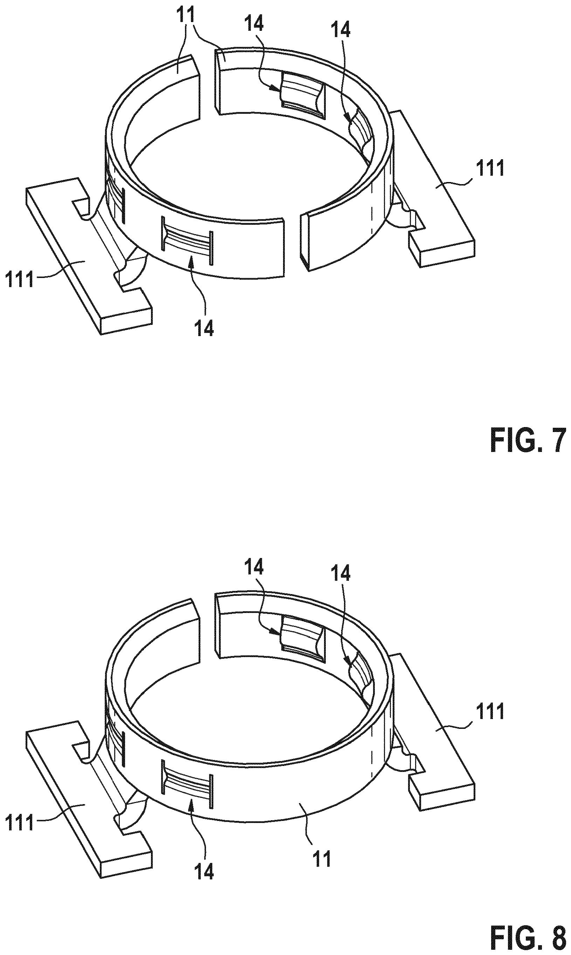

The first connector portion 1 includes a first contact element 11 and a pin receptacle 12. The first contact element 11 is a metallic element having an annular basic shape (such as a circular ring shape). The shape of the first contact element 11 will be explained in further detail below, e.g. with reference to figures 7 and 8. The pin receptacle 12 is formed as a hole extending through a metallic contact pad 15 of the first connector portion 1.

The first connector portion 1 is mounted on a printed circuit board (PCB) 41. Specifically, the contact pad 15 comprising the pin receptacle 12 may be mounted in a first side (upper side) 41-1 of the PCB 41, and the first contact element 11 may be mounted on a second side (lower side) 41-2 of the PCB 41, wherein the second side 41-2 of the PCB 41 is opposite to the first site 41-1 of the PCB 41.

The PCB 41 may form a part of an electronics module of the implantable medical device. In other words, Figure 1 shows only a portion of a larger PCB 41 of an electronics module of the implantable medical device. In the present embodiment, said portion of the PCB 41 of the electronics module is an extension of the PCB 41 having a circular portion, on which each of the first contact element 11 and the contact pad 15, which forms the pin receptacle 12, are mounted.

The contact pad 15, which includes the pin receptacle 12, is provided in the form of a surface-mount device (SMD) that is mounted on the first side 41-1 of the PCB 41. The first contact element 11 is also a part of an SMD, which is mounted on the second side 41-2 of the PCB 41. This will be explained in further detail below, e.g., with reference to Figures 2-4.

The second connector portion 2 comprises a second contact element 21 and a contact pin 22, which are arranged on the battery 42. The contact pin 22 axially protrudes from a housing of the battery 42 and has a positive potential (+). In other words, the contact pin 22 forms a first pole (+) of the battery 42. For example, the contact pin 22 may also extend in the interior of the battery 42, as will become apparent, e.g., from the cross-section view in Figure 2. The second contact element 21 is provided in the form of a circular metallic element which extends around the contact pin 22 inside a recess formed in the battery housing. The second contact element 21 has a negative potential, thus forming a second pole (-) of the battery 24.

While the connector arrangement 3 is depicted in a disconnected state in Fig. 1, it is configured to assume a connected state, in which the first contact element 11 is in contact with the second contact element 21 for connecting the first pole (+), and the contact pin 22 is received in (and in contact with) the pin receptacle 12 for connecting the second pole (-).

Figure 2 shows a cross-section view of the connector arrangement 3 of Figure 1. In Figure 2, the connector arrangement 3 is still in its disconnected state. In this cross-section view, further structural details regarding, e.g., the first connector portion 1, become apparent. For example, as illustrated in Figure 2, the first connection element 11 may be mounted on an insulation element 17. For example, the insulation element 17 may consist of a plastic material, such as an insulating mold compound. Thus, the insulation element 17 and the first connection element 11 may together form an SMD, which is mounted on the second side 41-2 of the PCB 41. For establishing an electrical connection with the PCB 41, the SMD further comprises bond feet 111, which are electrically connected with the first contact element 11. For example, the bond feet 111 may be formed in one (metallic) piece with the first contact elements.

The cross-section view in Figure 2 further reveals that the PCB 41 has a bore, which is axially aligned with the pin receptacle 12 of the contact pad 15, so as to allow for the contact pin 22 to extend through the bore and into the pin receptacle 12 in the connected state of the connector arrangement 3.

Regarding further structural properties of the first contact element 11, Figure 2 illustrates several second spring elements 14, which laterally protrude from the ring-shaped first contact element 11. The second contact element 21 exhibits a circumferential groove 24. In the connected state of the connector arrangement 3, the second spring elements 14 form-fittingly and force-fittingly engage with the groove 24 so as to secure a mechanical connection between the first contact element 11 and the second contact element 21. Hence, the first contact element 11 and the second contact and 21 are configured to be connected with each other by means of a spring connection or snap-in connection in the connected state.

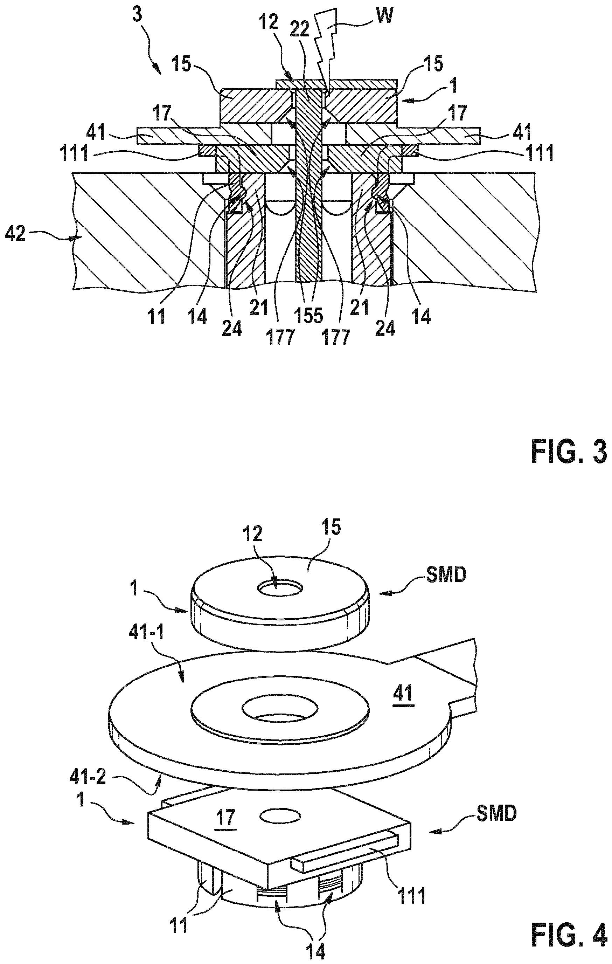

Figure 3 shows a cross-section view of the connector arrangement 3 of Figures 1 and 2 in its connected state (also referred to as mated condition). As illustrated, in the connected state, the contact pin 12 extends through the respective bores provided in the insulation element 17 and the PCB 41 into the pin receptacle 12. Thus, a contact between the contact pad 15 forming the pin receptacle 12 and the contact pin 22 is established. It should be noted that the insulation element 17 and the contact pad 15 comprise inclined insertion surfaces 177, 155, which are configured to guide the contact pin 22 into the bore of the insulation element 17 and the pin receptacle 12. In this way, a potential axial displacement between the insulation element 17, the PCB 41, and/or the contact pad 15 may be compensated to some extent.

Further, in the connected state, the first contact element 11 engages with the second contact element 21 so as to establish an electrical contact. Specifically, the second spring elements 14 engage with the groove 24 to secure the mechanical connection between the first contact element 11 and the second contact element 12, but also the connection between the first connector portion 1 and the second connector portion 2 as a whole.

In addition, the contact pin 22 may be materially bonded with the pin receptacle 12 in the connected state. Particularly, a metallurgical junction may be provided, e.g., by means of a welded joint, a soldered joint or a brazed joint. For example, the material junction may be created by means of a laser welding process W, as schematically indicated in Figure 3. For example, by fixing the contact pin 22 to the pin receptacle 12 (i.e. to the contact pad 15 forming the pin receptacle 12) by means of a welding spot or a soldering spot, an extraordinary contact reliability, may be ensured, which is generally required for medical implants. Further, due to said fixing of the contact pin 22 to the pin receptacle 12 by means of, e.g., a welded joint or a soldered joint, also the first contact element 11 may be held in contact with the second contact element 21. In other words, in addition to the spring connection formed by the second spring elements 14 and the groove 24, also the material bond between the contact pin 22 and the pin receptacle 12 may secure the connection between the first contact element 11 and the second contact element 21.

In accordance with the connector assembly 3 of Figures 1-3 (as well as all further embodiments explained below), the first connector portion 1 is directly connected with the PCB 41 instead of using an additional adapter. With this solution, the number of contact transitions may be reduced to two contact transitions for each pole. Specifically, in the connected state, the first contact element 11 is in contact with the second contact element 21 for connecting the first pole (-), and the contact pin 22 is be received in the pin receptacle 12 for connecting the second pole (+). In that case, only two interconnection transitions at each pole are sufficient: for the first pole (-), a transition from, the second contact element 21 arranged on the battery housing to the first contact element 11 and a transition from the first contact element 11 to a pad on the PCB 41, which is connected to the functional circuitry of the implantable medical device; and for the second pole (+), a transition from the contact pin 22 to the contact pad 15 of the first connector portion 1, and a transition from the contact pad 15 to the circuitry (e. g. via a corresponding pad on the PCB 41). In sum, only four contact transitions are required for the closed electric circuit which supplies the implantable medical device with energy.

Further, it should be noted that in the connected state as exemplarily illustrated in Figure 3, the first contact element 11, the second contact element 21, the contact pin 22, and the pin receptacle 12 may be arranged coaxially. This is to say that each of the first contact element 11, the second contact element 21, the contact pin 22, and the pin receptacle 12 may be arranged coaxially with respect to a common symmetry axis. For example, said common symmetry axis may be defined by a main extension axis of the contact pin 22 (pointing in the vertical direction in Fig. 3) as well as by a (virtual) axis of the annular basic shape of the first contact element 11 and/or the second contact element 21. The connector arrangement 3 may be very volume efficient (i.e. space saving) due to such a coaxial arrangement of its components.

Figure 4 shows an exploded view of the first connector portion 1 of the connector arrangement 3 in combination with the PCB 41. This view clearly shows the bore that is provided in the PCB 41 for letting pass through the contact pin 12 in the connected state. Further, Figure 4 illustrates an upper side of the insulation element 17 including the bond feet 111.

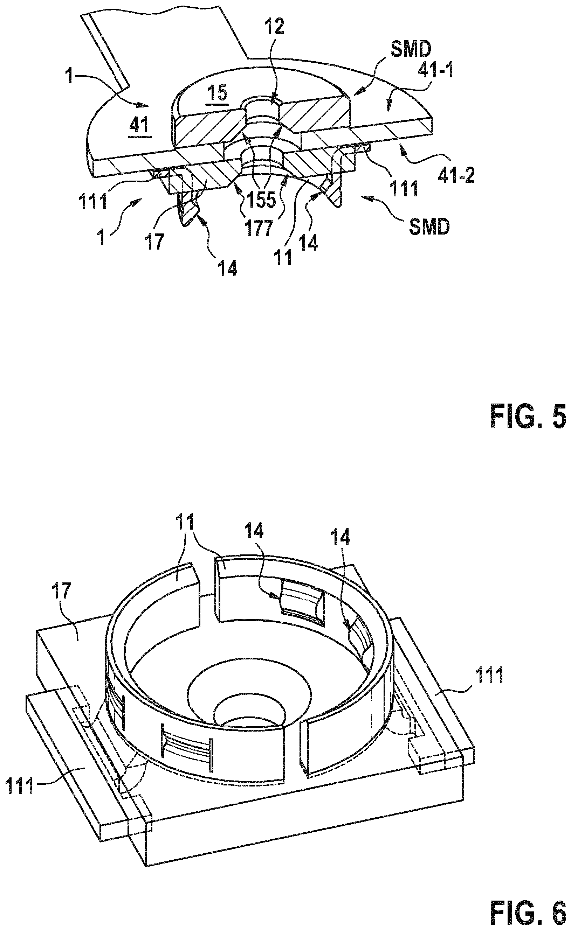

Figure 5 shows a cross-section view of the first connector portion 1 of Figure 4 in an assembled state, i. e., in a state, wherein both surface-mount devices (the contact pad 15 as well as the insulation element 17 together with the first contact element 11) are coaxially mounted on a respective side of the PCB 41. For example, the assembly of the SMDs may comprise a corresponding reflow soldering steps, in which the SMDs are attached to the PCB 4. The cross-section view of Figure 5 also illustrates the inclined insertion surfaces 155, 177 mentioned above.

Figure 6 shows a perspective view of the first contact element 11 arranged on the insulation element 17. The metallic first contact element 11 including the second spring elements 14 may have a plating that comprises, for example, at least one of the following materials: phosphorus-bronze, nickel, and gold. For example, in a preferred embodiment, the first contact element 11 is provided with an electroless nickel inversion gold (ENIG) plating.

The insulation element 17 may comprise a molded plastic body, which may comprise or consist of one or more liquid-crystal polymers (LCP), for example. For instance, the first contact element 11 may be partially molded in the insulation element 17.

Figure 7 shows a perspective view of the first contact element 11 of Figure 6. In this exemplary embodiment, the first contact element 11 consist of two separate, basically semicircular elements, wherein each of these elements is formed in one piece with a respective bond foot 111.

Figure 8 shows a perspective view of another exemplary variant of the first (female) contact element 11. In this case, the first contact element 11, including the second spring elements 14, is formed in one piece, which also includes two solderable bond feet 111. The bond feed 111 are provided for soldering the first contact element 11 to the PCB 41.

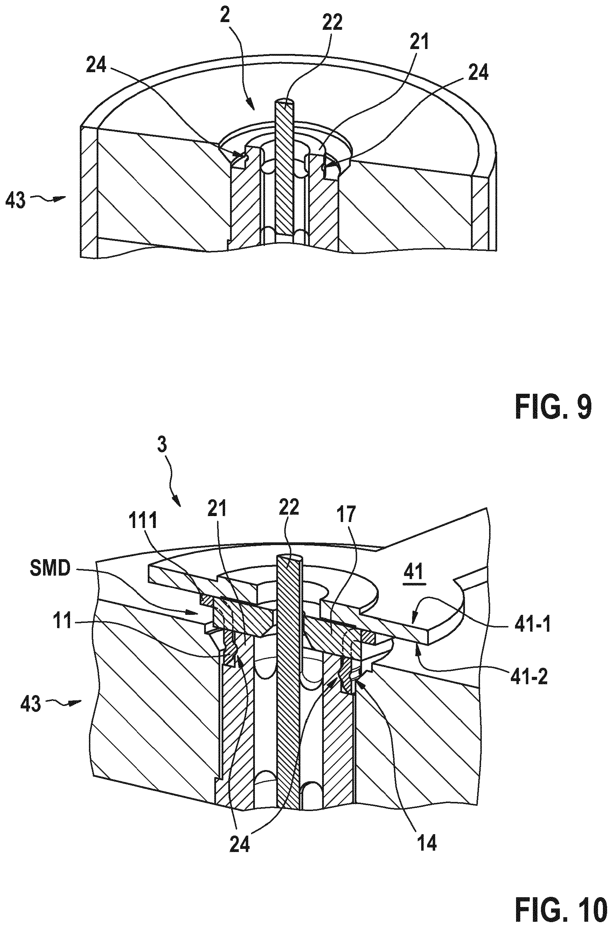

In Figure 9, a second connector portion 2 is provided on a feedthrough assembly 43 (instead of the battery 42 of Figures 1-3). The term "feedthrough" refers to a component that hermetically seals a conductive pin coming out of the inner assembly of the battery from the exterior (in that case the device electronics). The electrically conductive pin transmits electrical energy to the device electronics. For example, the second contact element 21 may be plated with gold or another plating material. In the exemplary embodiment shown in Figure 9, the second contact element 21 is provided inside a recess formed in a housing of the feedthrough assembly 43, similar to what has that been described above with respect to Figures 1 and 2. As a result, the second contact element 21 does not extend beyond an outer contour of the housing of the feedthrough assembly 42. This is a very volume efficient (i.e., space saving) design.

Figure 10 shows a cross-section view of the second (male) connector portion 2 of Figure 9, which is coaxially connected with a part of a first connector portion 1. What has been stated above with respect to the first connector portion 1 also applies to the first connector portion 1 of the present embodiment. In particular, in Figure 10, the first contact element 11 is force-fittingly and form-fittingly connected with the second contact element 21 by means of a spring connection or snap-in connection, as described above. Further, the contact pin 22 extends through bores provided in the insulation element 17 and the PCB 41, respectively. The contact pad 15, which forms the pin receptacle 12, is not shown in Figure 10.

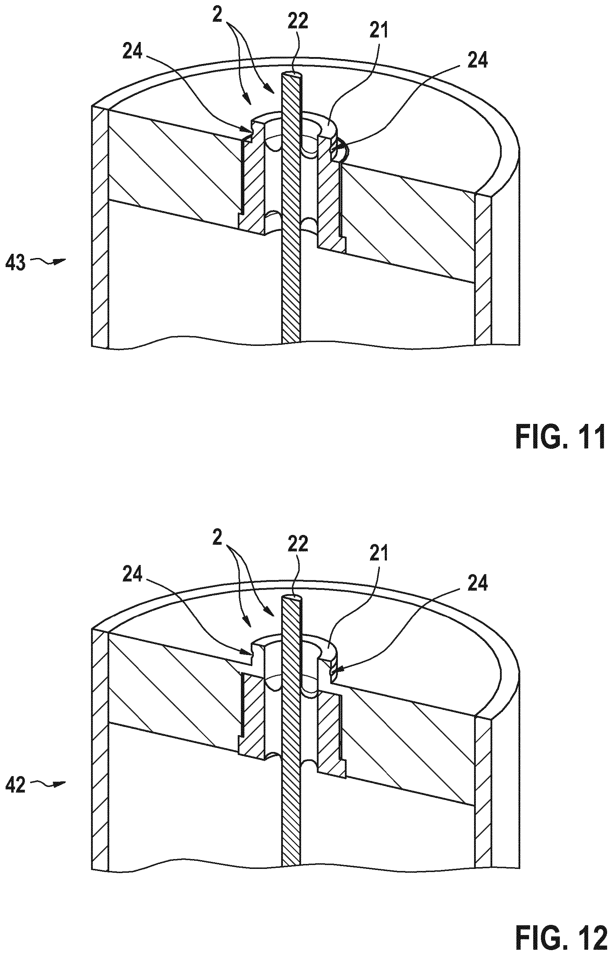

Figure 11 shows a cross-sectional view of another variant of a second connector portion 2. In this exemplary variant, the second contact element 21 is integrated in the housing of the feedthrough assembly 43. Further, the second contact element 21 axially protrudes from a frontend server surface of the housing of the feedthrough assembly 43. In other words, the second conductive 21 defines a portion of an outer contour of the housing of the feedthrough assembly 43.

Figure 12 shows a cross-section view of yet another variant of second connector portion 2. In this case, the second connector portion 2 is arranged on a battery 42. Apart from that, the arrangement is very similar to the one described above with reference to Figure 11. In particular, the second contact element 21 is integrated in a housing of the battery 42 and axially protrudes from a front surface of said housing.

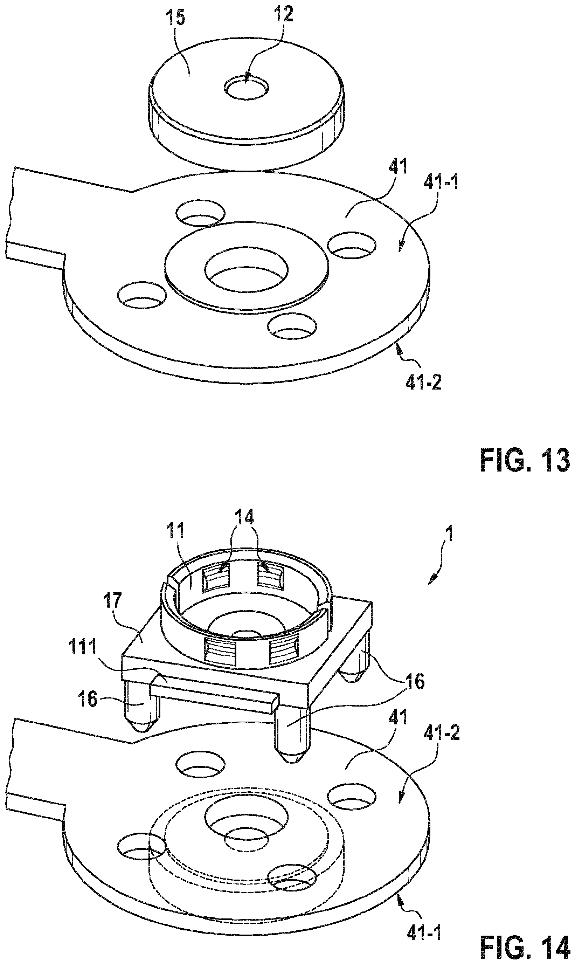

Figure 13 shows a perspective view of a PCB 41 and a contact pad 15 forming a pin receptacle 12, wherein the contact pad 15 is not (yet) mounted on the front side 41-1 of the PCB 41. In this exemplary embodiment, the PCB 41 comprises four alignment bores that are arranged around a central bore that is provided so as to let pass through the connection pin 22 of the second connector portion 2 in the connected state of the connector arrangement 3.

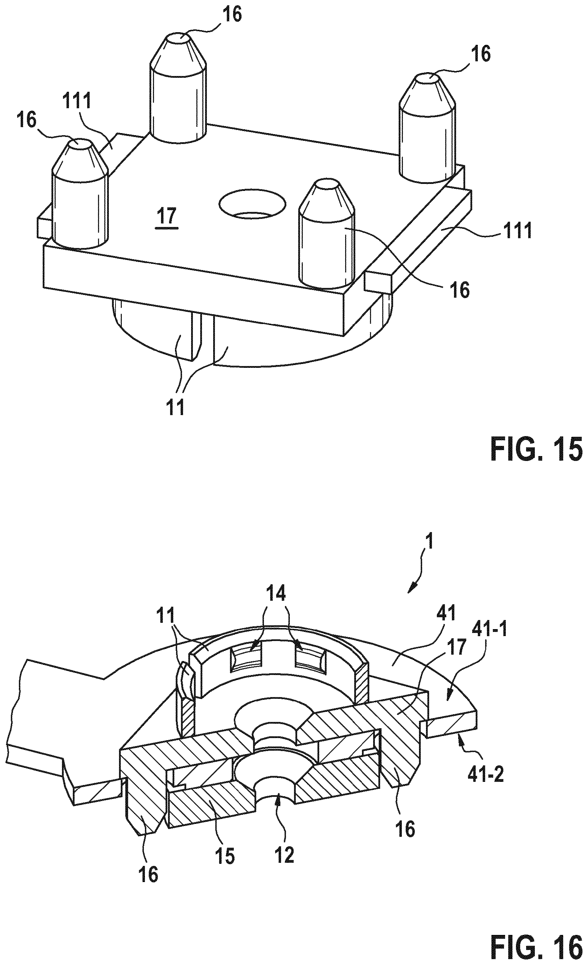

Figure 14 shows the backside 41-2 of the PCB 41 of Figure 13, as well as an SMD formed by a first contact element 11 that is arranged on an insulation element 17. The insulation element 17 comprises four guidance elements 16, which protrude from the four corners of the insulation element 17. Figure 15 shows a close-up perspective view of the first contact element 11 and the insulation element 17 including the guidance elements 16.

Figure 16 shows a cross-section view of the PCB 41, the contact pad 15, and the insulation element 17 of Figures 13-15 in an assembled state. In the assembled state, each of the insulation element 17 and the contact pad 15 are soldered to a respective side of the PCB 41. As illustrated, in the assembled state, the guidance elements 16 extend through the alignment bores provided in the PCB 41. Thus, the guidance elements 16 (in connection with the alignment bores provided in the PCB 41) are configured to axially align the contact element 11, the PCB 41, and the contact pad 15 that forms the pin receptacle 12, e.g., during the reflow soldering processes, which attach the insulation element 17 and the contact pad 15 to the PCB 41.

For example, during the reflow soldering process, when each of the contact pad 15 and the insulation element 17 "swim" in the solder for a short while before the solder then solidifies during cooling off, there is a risk that an axial offset between these contact components arises. This offset may be partially compensated for by means of the inclined insertion surfaces 155, 177, which may guide the contact pin towards the hole (i.e., the pin receptacle 12) formed in the contact pad 15. To further minimize such position tolerances, the guidance elements 16 are arranged at the insulation element 17 so as to ensure an axial alignment and avoid an unwanted displacement during the reflow process (i.e., while the solder is liquid).

For example, the guidance elements 16 may laterally engage with a circumferential edge of the contact pad 15 so as to establish said axial alignment, as illustrated in Figure 16. In the illustrated exemplary embodiment, the guidance elements 16 extend through alignment bores provided in the PCB 41. In an alternative embodiment, which is not illustrated, a portion of the PCB 41 may be smaller than the insulation element 17 such that the guidance elements 16 may laterally engage with a circumferential edge of said portion of the PCB 41 instead of extending through dedicated alignment bores provided in the PCB 41. Further, in an alternative embodiment (also not illustrated), the contact pad 15 may comprise dedicated alignment bores through which the guidance elements 16 may extend upon assembly of the first connector portion 1.

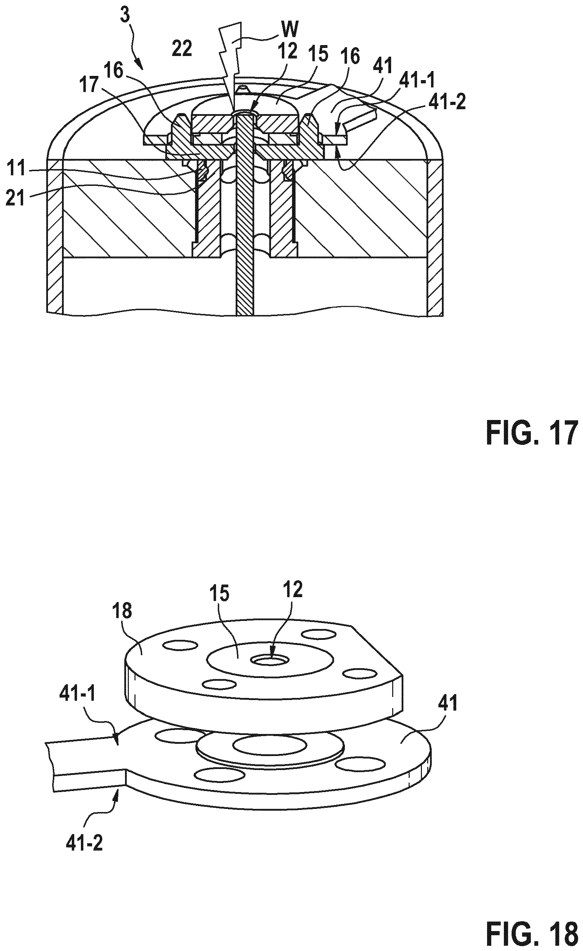

Figure 17 shows a cross-section view of the first connector portion 1 of Figure 16, which is connected to a second connector portion 2 of a battery 42. As already described above with reference to Figure 3, in the mated condition, the contact pin 22 may be materially bonded with the pin receptacle 12, e.g., by means of a laser welding process W.

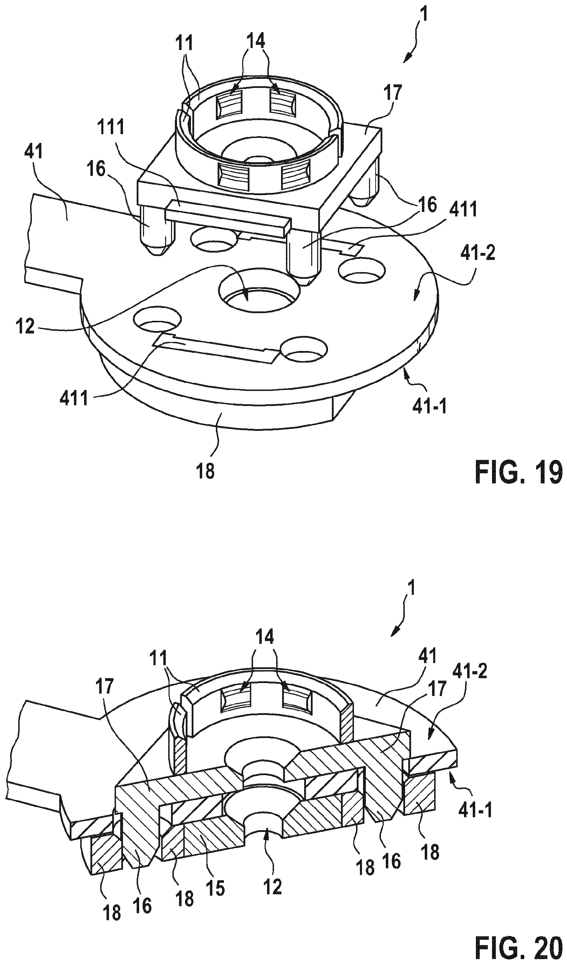

Figure 18 shows another variant of a PCB 41 and a contact pad 15 forming a pin receptacle 12. In this embodiment, the contact pad 15 is surrounded by a ring-shaped plastic border 18, wherein the plastic border 18 comprises four alignment bores matching the alignment bores of the PCB 41. Figure 19 shows the components already shown in Figure 18 and, in addition, a first contact element 11 that is arranged on an insulation element 17. The insulation element 17 has four guidance elements 16, as described above with reference to, e.g., Figures 14-17. In Figure 19, bond pads 11 are illustrated, which are configured to establish an electrical connection with the bond feet 111 when the SMD comprising the insulation element 17 and the first contact element 11 is fitted to the second side 41-2 of the PCB 41.

Figure 20 shows the components of Figure 19 is in an assembled state, wherein the guidance bores and that elements 16 extend into the alignment bores of the plastic border 18. Thus, an exact concentric alignment of the central bores in the insulation element 17 and the contact pad 15 may be achieved. It should be noted that an alternative embodiment, which is not illustrated in the figures, the plastic border 18 of the contact pad 15 (instead of the insulation element 17) may be provided with protruding guidance elements, whereas the insulation 17 (instead of the plastic border 18) may have corresponding alignment bores.

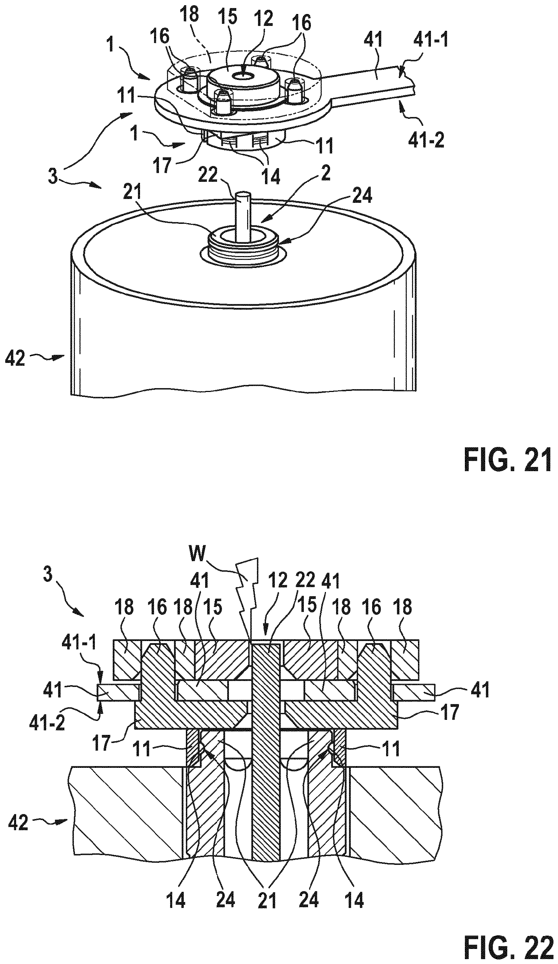

Figure 21 shows a perspective view of the first connector portion 1 of Figure 20 and a battery 42 having a second contact portion 2 as described above in connection with, e.g., Figure 12. In the situation depicted in figure 21, the connector assembly 3 is in a disconnected state. Figure 22 shows a cross-section view of the connector arrangement 3 of Figure 21 in the connected state, wherein the connector pin is metallurgically bonded with the contact pad 15 that forms the pin receptacle 12. The metallurgical bond is created, e.g., by means of a laser welding process W.

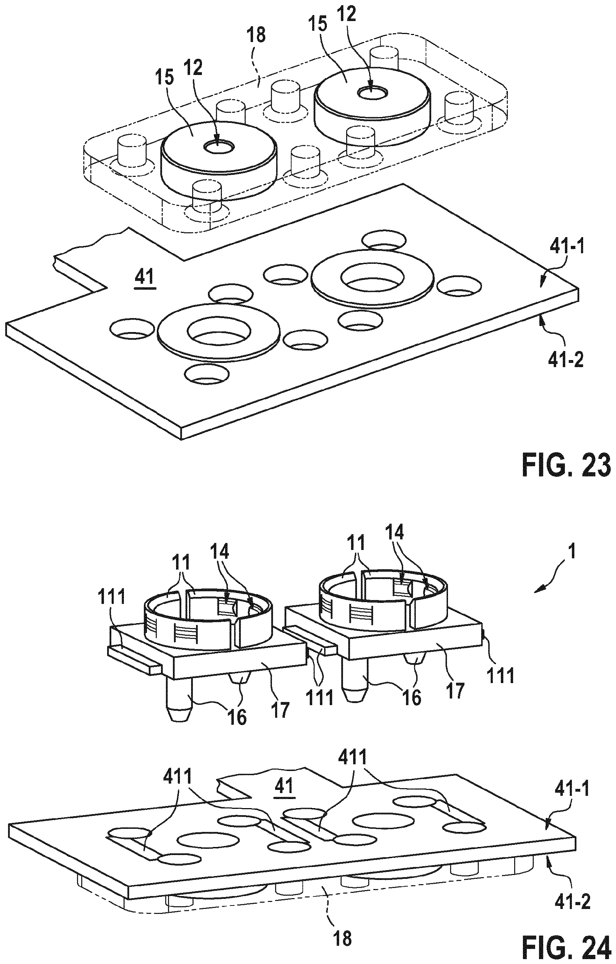

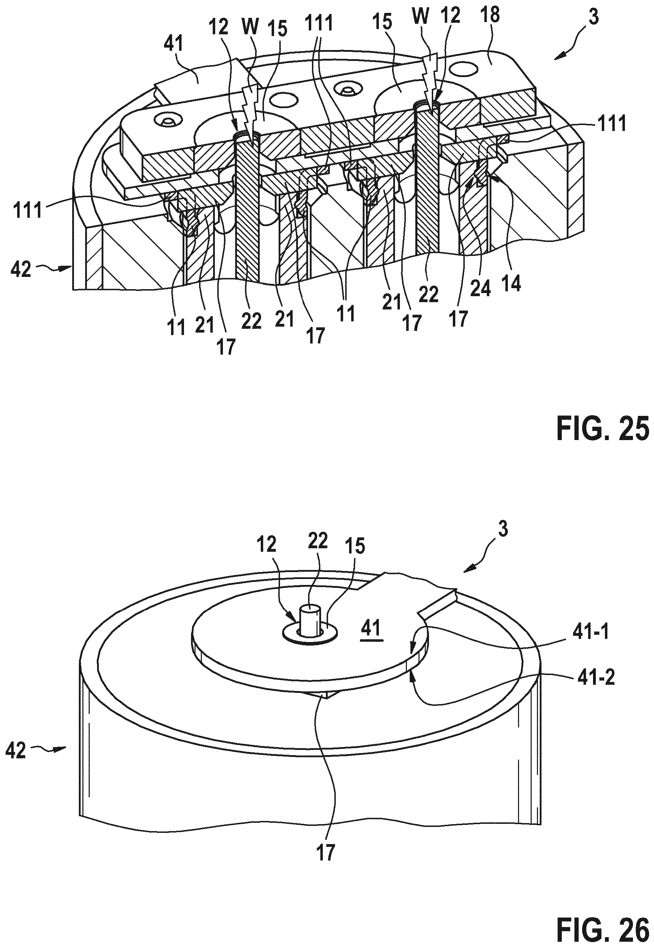

Figure 23 shows a perspective view of a PCB 41 and an array of two contact pads 15, each forming a respective pin receptacle 12. In this exemplary embodiment, the two contact pads 15 are surrounded (and connected to each other) by a common plastic border 18. The PCB 41 comprises a total of eight alignment bores. Correspondingly, eight alignment bores are also provided in the plastic border 18. Figure 24 shows the PCB 41 and the contact pad array 15 of Figure 23 as well as two first contact elements 11 that are arranged on respective insulation elements 17. Figure 25 shows a cross-section view of the first connector portion 1 of Figure 24 that is connected to a second connector portion 2 of a battery 42. Similar to what has been described above, also in this embodiment, which uses an array of two pin receptacles 12 and two corresponding contact pins 22, each contact pin 22 may be welded to the respective contact pad 15 in the mated condition of the connector arrangement 3. For example corresponding welding spots may be created by means of a laser welding process W.

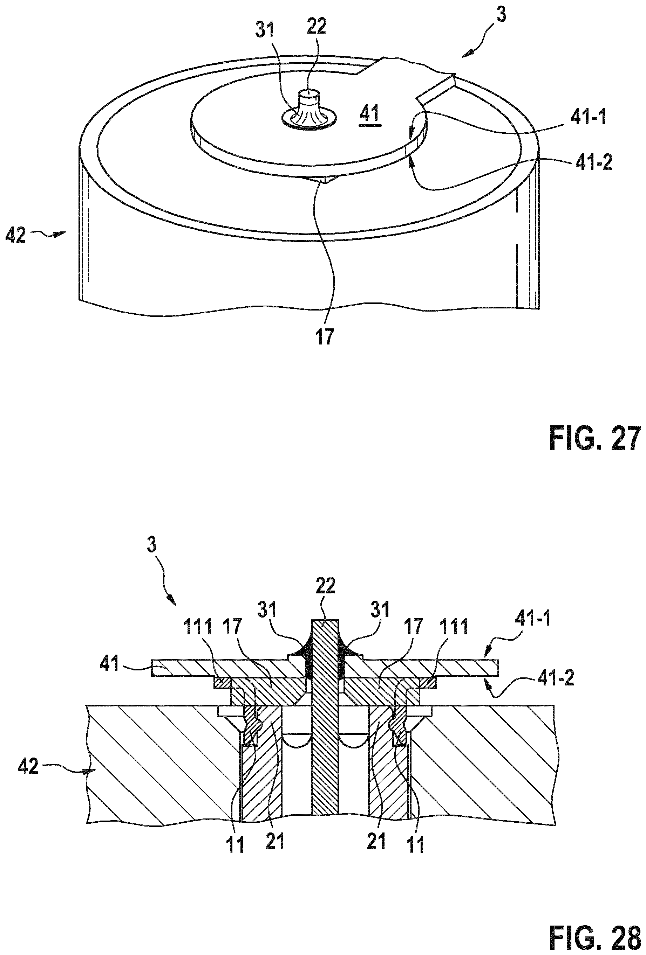

Figure 26 shows a perspective view of yet a further variant of a connector arrangement 3 in the connected state. Here, the contact pad 15 is arranged directly on the PCB 41 and has a ring-shaped solderable surface that surrounds the pin receptacle 12. In the connected state, a distal end of the contact pin 22 extends through the pin receptacle 12 and axially protrudes above the PCB 41 and the solderable surface of the contact pad 15. For example, the contact pin 22 may be plated, e.g., with gold, palladium or ENIG, so as to enable soldering. Figure 27 shows the connector arrangement of Figure 26, wherein the contact pin 22 is soldered to the pin receptacle 12 (see the soft solder 31). In other words, in this exemplary embodiment, the material bond between the connector pin 22 and the pin receptacle 12 is created by means of a soft solder process. This is further illustrated in Figure 28, which shows a cross-section view of the connector arrangement 3 of Figure 27.

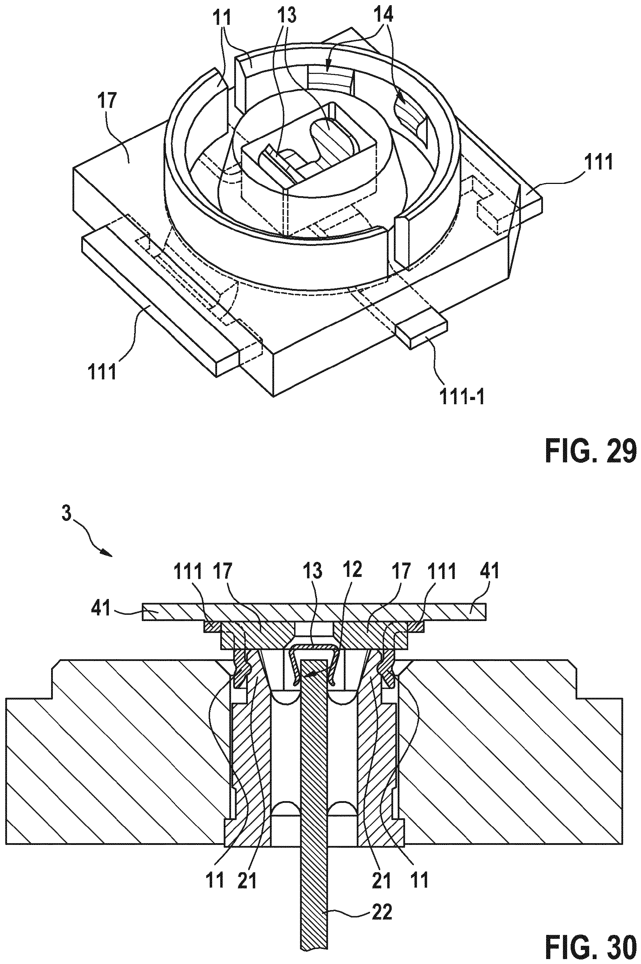

Figure 29 shows another variant of a first connector portion 1, wherein the pin receptacle 12 is formed by a first spring element 13. In this embodiment, the first spring element 13 is configured to clamp the distal end of the contact pin 22 in the connected state, thereby establishing a form-fitting and force fitting connection between the pin receptacle 12 and the contact pin 22. For example, the first spring element 13 is connected with one or more solderable bond feet 111-1. The first spring element 13 and the bond feet 111-1 that are illustrated in Figure 29 may also be formed in one piece. For example, the material and/or plating of the first spring element 13 and/or of the bond feet 111-1 may be the same as the material and/or of the first contact element 11 (which may have, e.g., a plating comprising phosphorous, bronze, nickel, gold or ENIG, as explained above).

Figure 30 shows a cross-section view of the first connector portion 1 of Figure 29 that is connected with a second connector portion 2. In the connected state of this connector assembly 3, the contact pin 22 is clamped inside the first spring element 13. It should be noted that here, the contact pin 22 does not extend through the PCB 41 in the connected state. The first contact element 11 and the second contact element 21 are connected by means of a spring connection or snap-in connection, as described above.

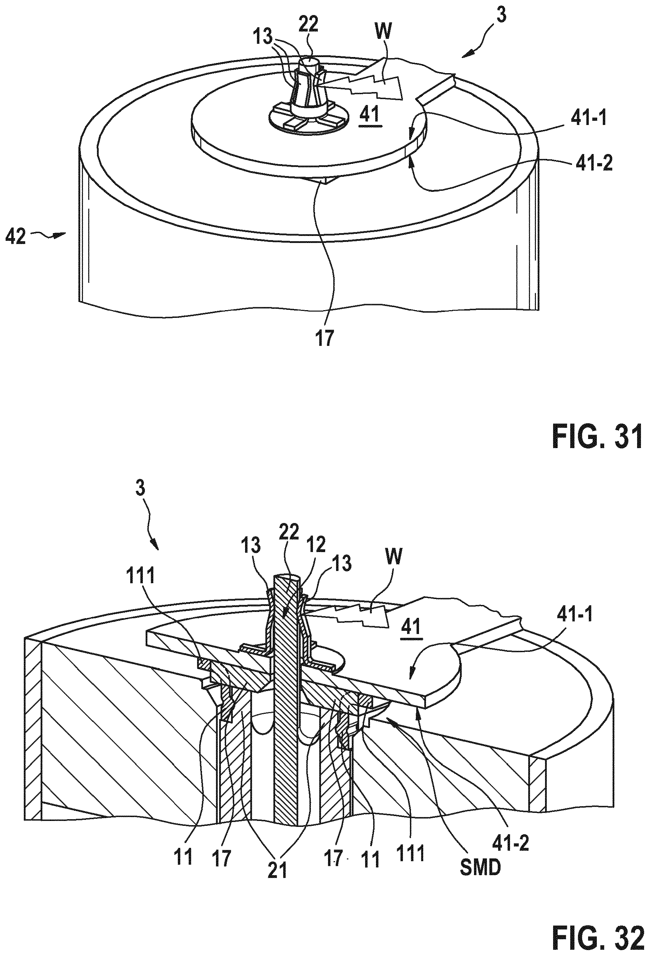

Figure 31 shows yet another variant of a connector assembly 3 having a first spring connection 13 for securing the contact pin 22 in the connected position. here, the first spring connection 13 is arranged on the contact pad 15, i.e., on the first side 41-1 of the PCB 41. In the connected state, the distal end of the contact pin 22 extends through the PCB and is clamped by the first spring element 13. Optionally, a metallurgical bond may be created between the first spring element 13 and the contact pin 22, e. g., by means of a laser welding process W. This embodiment is further illustrated in Figure 32, which shows a cross-section view of the connector assembly 3 of Figure 31.

### LIST OF REFERENCE NUMERALS

- 1: First connector portion
- 11: First contact element
- 111: Bond foot
- 111-1: Bond foot
- 12: Pin receptacle
- 13: First spring element
- 14: Second spring element
- 15: Contact pad
- 155: Insertion surface
- 16: Guidance element
- 17: Insulation element
- 177: Insertion surface
- 18: Plastic border
- 2: Second connector portion
- 21: Second contact element
- 22: Contact pin
- 24: Groove
- 3: Connector arrangement
- 31: Soft solder
- 4: Implantable medical device
- 41: Printed circuit board
- 411: Bond pads
- 41-1: First side
- 41-2: Second side
- 42: Battery
- 43: Passage
- SMD: Surface-mount device
- W: Welding process

## Claims

1. An implantable medical device having a connector arrangement (3) for establishing a two-pole electric contact between components of the implantable medical device (4), the connector arrangement (3) comprising
a first connector portion (1) comprising a first contact element (11) and a pin receptacle (12); and
a second connector portion (2) comprising a second contact element (21) and a contact pin (22); and
a printed circuit board (41)
wherein, the connector arrangement (3) is configured to assume a connected state, in which
the first contact element (11) is in contact with the second contact element (21); and
the contact pin (22) is received in the pin receptacle (12);
wherein at least a part of the first connector portion (1) or at least a part of the second connector portion (2) is mounted on the printed circuit board (41).

2. The implantable medical device of claim 1, wherein the first connector portion (1) or the second connector portion (2) forms at least a part of a surface-mount device (SMD) that is mounted on the printed circuit board (41).

3. The implantable medical device of one of the preceding claims, wherein the pin receptacle (12) is mounted on a first side (41-1) of the printed circuit board (41) and the first contact element (11) is mounted on a second side (41-2) of the printed circuit board (41) that is opposite to the first side (41-1).

4. The implantable medical device of one of the preceding claims, wherein the connector arrangement (3) is in the connected state and the contact pin (22) is materially bonded with the pin receptacle (12).

5. The implantable medical device of claim 4, wherein the contact pin (22) is bonded with the pin receptacle (12) by means of at least one of: a welded joint, a soldered joint, and a brazed joint.

6. The implantable medical device of one of the preceding claims, wherein the connector arrangement (3) is in the connected state and the contact pin (22) is form-fittingly and/or force-fittingly connected to the pin receptacle (12).

7. The implantable medical device of one of the preceding claims, wherein the first connector portion (1) comprises a first spring element (13) that is configured to establish a form-fitting and/or force-fitting connection between the pin receptacle (12) and the contact pin (22) in the connected state of the connector arrangement (1).

8. The implantable medical device of one of the preceding claims, wherein the first contact element (11) and the second contact element (21) are configured to be connected with each other by means of a spring connection and/or by means of a snap-in connection in the connected state of the connector arrangement (3).

9. The implantable medical device of claim 8, wherein one of the first contact element (11) and the second contact element (21) comprises at least one second spring element (14) and the other one of the first contact element (11) and the second contact element (21) comprises at least one groove (24), wherein the at least one second spring element (14) engages with the at least one groove (24) in the connected state of the connector arrangement (3).

10. The implantable medical device of one of the preceding claims, wherein the first contact element (11) and/or the second contact element (21) have an annular basic shape.

11. The implantable medical device of one of the preceding claims, wherein each of the first contact element (11), the second contact element (21), the contact pin (22), and the pin receptacle (12) are arranged coaxially with respect to a common symmetry axis in the connected state of the connector arrangement (3).

12. The implantable medical device of one of the preceding claims, wherein the first connector portion (1) comprises a contact pad (15).

13. The implantable medical device of claim 12, wherein the first connector portion (1) comprises at least one guidance element (16) configured to align the first contact element (11) and the contact pad (15).

14. The implantable medical device of one of the claims 2 to 13, wherein the surface-mount device (SMD) is mounted on a printed circuit board (41) that is a part of an electronics module of the implantable medical device (4).

15. The implantable medical device of claim 14, wherein the first connector portion (1) is mounted on the printed circuit board (41) and the second connector portion (2) is arranged on a battery (42) or on a capacitor of the implantable medical device (4).

## Patentansprüche

1. Implantierbares medizinisches Gerät mit einer Steckverbindungsanordnung (3) zur Herstellung eines zweipoligen elektrischen Kontakts zwischen Komponenten des implantierbaren medizinischen Geräts (4), die Steckverbindungsanordnung (3) umfassend
einen ersten Steckverbindungsabschnitt (1), ein erstes Kontaktelement (11) und eine Stiftaufnahme (12) umfassend; und
einen zweiten Steckverbindungsabschnitt (2), ein zweites Kontaktelement (21) und einen Kontaktstift (22) umfassend; und
eine Leiterplatte (41)
wobei die Steckverbindungsanordnung (3) dafür ausgelegt ist, einen gesteckten Zustand anzunehmen, bei dem
das erste Kontaktelement (11) mit dem zweiten Kontaktelement (21) in Kontakt ist; und
der Kontaktstift (22) in der Stiftaufnahme (12) Aufnahme findet;
wobei mindestens ein Teil des ersten Steckverbindungsabschnitts (1) oder mindestens ein Teil des zweiten Steckverbindungsabschnitts (2) auf der Leiterplatte (41) montiert ist.

2. Implantierbares medizinisches Gerät nach Anspruch 1, wobei der erste Steckverbindungsabschnitt (1) oder der zweite Steckverbindungsabschnitt (2) mindestens einen Teil eines oberflächenmontierten Bauelements (SMD) bildet, das auf der Leiterplatte (41) montiert ist.

3. Implantierbares medizinisches Gerät nach einem der vorstehenden Ansprüche, wobei die Stiftaufnahme (12) an einer ersten Seite (41-1) der Leiterplatte (41) montiert ist und das erste Kontaktelement (11) an einer zweiten Seite (41-2) der Platine (41) montiert ist, die der ersten Seite (41-1) gegenüber liegt.

4. Implantierbares medizinisches Gerät nach einem der vorstehenden Ansprüche, wobei die Steckverbindungsanordnung (3) im gesteckten Zustand ist und der Kontaktstift (22) stoffschlüssig mit der Stiftaufnahme (12) verbunden ist.

5. Implantierbares medizinisches Gerät nach Anspruch 4, wobei der Kontaktstift (22) mit der Stiftaufnahme (12) mittels mindestens eines von Folgendem verbunden ist: einer Schweißstelle, einer weichgelöteten Stelle und einer hartgelöteten Stelle.

6. Implantierbares medizinisches Gerät nach einem der vorstehenden Ansprüche, wobei die Steckverbindungsanordnung (3) im gesteckten Zustand ist und der Kontaktstift (22) formschlüssig und/oder kraftschlüssig mit der Stiftaufnahme (12) verbunden ist.

7. Implantierbares medizinisches Gerät nach einem der vorstehenden Ansprüche, wobei der erste Steckverbindungsabschnitt (1) ein erstes Federelement (13) umfasst, das dafür ausgelegt ist, eine formschlüssige und/oder kraftschlüssige Verbindung zwischen der Stiftaufnahme (12) und dem Kontaktstift (22) im gesteckten Zustand der Steckverbindungsanordnung (3) herzustellen.

8. Implantierbares medizinisches Gerät nach einem der vorstehenden Ansprüche, wobei das erste Kontaktelement (11) und das zweite Kontaktelement (21) dafür ausgelegt sind, miteinander mittels einer Federverbindung und/oder eines Rastanschlusses im gesteckten Zustand der Steckverbindungsanordnung (3) verbunden zu werden.

9. Implantierbares medizinisches Gerät nach Anspruch 8, wobei eins vom ersten Kontaktelement (11) und vom zweiten Kontaktelement (21) mindestens ein zweites Federelement (14) und das andere des ersten Kontaktelements (11) und des zweiten Kontaktelements (21) mindestens eine Rille (24) umfasst, wobei das mindestens eine zweite Federelement (14) im gesteckten Zustand der Steckverbindungsanordnung (3) mit der mindestens einen Rille (24) ineinandergreift.

10. Implantierbares medizinisches Gerät nach einem der vorstehenden Ansprüche, wobei das erste Kontaktelement (11) und/oder das zweite Kontaktelement (21) eine ringförmige Grundform haben.

11. Implantierbares medizinisches Gerät nach einem der vorstehenden Ansprüche, wobei jedes des ersten Kontaktelements (11), des zweiten Kontaktelements (21), des Kontaktstifts (22) und der Stiftaufnahme (12) in Bezug auf eine gemeinsame Symmetrieachse im gesteckten Zustand der Steckverbindungsanordnung (3) koaxial angeordnet ist.

12. Implantierbares medizinisches Gerät nach einem der vorstehenden Ansprüche, wobei der erste Steckverbindungsabschnitt (1) ein Kontakt-Pad (15) aufweist.

13. Implantierbares medizinisches Gerät nach Anspruch 12, wobei der erste Steckverbindungsabschnitt (1) mindestens ein Führungselement (16) umfasst, das dafür ausgelegt ist, das erste Kontaktelement (11) und das Kontakt-Pad (15) aufeinander auszurichten.

14. Implantierbares medizinisches Gerät nach einem der Ansprüche 2 bis 13, wobei das oberflächenmontierte Bauelement (SMD) auf einer Leiterplatte (41) montiert ist, die Teil eines Elektronikmoduls des implantierbaren medizinischen Geräts (4) ist.

15. Implantierbares medizinisches Gerät nach Anspruch 14, wobei der erste Steckverbindungsabschnitt (1) auf der Leiterplatte (41) montiert und der zweite Steckverbindungsabschnitt (2) auf einer Batterie (42) oder einem Kondensator des implantierbaren medizinischen Geräts (4) angeordnet ist.

## Revendications

1. Dispositif médical implantable possédant un agencement de connecteurs (3) permettant d'établir un contact électrique bipolaire entre des composants du dispositif médical implantable (4), l'agencement de connecteurs (3) comprenant
un premier segment de connecteur (1) comprenant un premier élément de contact (11) et un réceptacle à broche (12) ; et
un deuxième segment de connecteur (2) comprenant un deuxième élément de contact (21) et une broche de contact (22) ; et
une carte de circuit imprimé (41)
dans lequel l'agencement de connecteurs (3) est conçu pour assurer un état connecté, par lequel
le premier élément de contact (11) est en contact avec le deuxième élément de contact (21) ; et
la broche de contact (22) est réceptionnée dans le réceptacle à broche (12) ;
dans lequel au moins une partie du premier segment de connecteur (1), ou au moins une partie du deuxième segment de connecteur (2), est montée sur la carte de circuit imprimé (41).

2. Dispositif médical implantable selon la revendication 1, dans lequel le premier segment de connecteur (1), ou le deuxième segment de connecteur (2), forme au moins une partie d'un dispositif de montage en surface (SMD) qui est monté sur la carte de circuit imprimé (41).

3. Dispositif médical implantable selon l'une des revendications précédentes, dans lequel le réceptacle à broche (12) est monté sur un premier côté (41-1) de la carte de circuit imprimé (41) et le premier élément de contact (11) est monté sur un deuxième côté (41-2) de la carte de circuit imprimé (41) qui est opposé au premier côté (41-1).

4. Dispositif médical implantable selon l'une des revendications précédentes, dans lequel l'agencement de connecteurs (3) est dans l'état connecté et la broche de contact (22) est liée matériellement avec le réceptacle à broche (12).

5. Dispositif médical implantable selon la revendication 4, dans lequel la broche de contact (22) est reliée avec le réceptacle à broche (12) au moyen d'au moins un élément parmi : un j oint amalgamé, un j oint soudé et un joint brasé.

6. Dispositif médical implantable selon l'une des revendications précédentes, dans lequel l'agencement de connecteurs (3) est dans l'état connecté et la broche de contact (22) est connectée par complémentarité des formes et/ou par complémentarité des forces au réceptacle à broche (12).

7. Dispositif médical implantable selon l'une des revendications précédentes, dans lequel le premier segment de connecteur (1) comprend un premier élément de ressort (13) qui est conçu pour établir une connexion par complémentarité des formes et/ou par complémentarité des forces entre le réceptacle à broche (12) et la broche de contact (22) dans l'état connecté de l'agencement de connecteurs (3).

8. Dispositif médical implantable selon l'une des revendications précédentes, dans lequel le premier élément de contact (11) et le deuxième élément de contact (21) sont conçus pour être connectés l'un avec l'autre au moyen d'une connexion par ressort, et/ou au moyen d'une connexion encliquetable, dans l'état connecté de l'agencement de connecteurs (3).

9. Dispositif médical implantable selon la revendication 8, dans lequel l'un parmi le premier élément de contact (11) et le deuxième élément de contact (21) comprend au moins un deuxième élément de ressort (14) et l'autre parmi le premier élément de contact (11) et le deuxième élément de contact (21) comprend au moins une rainure (24), dans lequel l'au moins un deuxième élément de ressort (14) se met en prise avec l'au moins une rainure (24) dans l'état connecté de l'agencement de connecteurs (3).

10. Dispositif médical implantable selon l'une des revendications précédentes, dans lequel le premier élément de contact (11) et/ou le deuxième élément de contact (21) ont une forme basique annulaire.

11. Dispositif médical implantable selon l'une des revendications précédentes, dans lequel chacun parmi le premier élément de contact (11), le deuxième élément de contact (21), la broche de contact (22) et le réceptacle à broche (12) sont agencés co-axialement par rapport à un axe de symétrie commun dans l'état connecté de l'agencement de connecteurs (3).

12. Dispositif médical implantable selon l'une des revendications précédentes, dans lequel le premier segment de connecteur (1) comprend un plot de contact (15).

13. Dispositif médical implantable selon la revendication 12, dans lequel le premier segment de connecteur (1) comprend au moins un élément de guidage (16) conçu pour aligner le premier élément de contact (11) et le plot de contact (15).

14. Dispositif médical implantable selon l'une des revendications 2 à 13, dans lequel le dispositif de montage en surface (SMD) est monté sur une carte de circuit imprimé (41) qui est une partie d'un module électronique du dispositif médical implantable (4).

15. Dispositif médical implantable selon la revendication 14, dans lequel le premier segment de connecteur (1) est monté sur la carte de circuit imprimé (41) et le deuxième segment de connecteur (2) est agencé sur une pile (42) ou sur un condensateur du dispositif médical implantable (4).
